(19) Europäisches Patentamt
European Patent Office
Office européen des brevets

(11) **EP 3 799 789 A1**

(12) **EUROPEAN PATENT APPLICATION**
published in accordance with Art. 153(4) EPC

(43) Date of publication:
**07.04.2021 Bulletin 2021/14**

(51) Int Cl.:
***A61B 6/00*** (2006.01)    ***G06T 7/00*** (2017.01)
***G06T 7/11*** (2017.01)

(21) Application number: **19848078.2**

(22) Date of filing: **19.06.2019**

(86) International application number:
**PCT/JP2019/024229**

(87) International publication number:
**WO 2020/031515 (13.02.2020 Gazette 2020/07)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **10.08.2018 JP 2018151967**

(71) Applicant: **CANON KABUSHIKI KAISHA OHTA-KU**
**Tokyo 146-8501 (JP)**

(72) Inventor: **TAKAHASHI, Naoto**
**Tokyo 146-8501 (JP)**

(74) Representative: **WESER & Kollegen**
**Patentanwälte PartmbB**
**Radeckestraße 43**
**81245 München (DE)**

(54) **IMAGE PROCESSING DEVICE, IMAGE PROCESSING METHOD, AND PROGRAM**

(57)    An image processing apparatus divides a radiological image obtained by performing radiography on a subject, into a plurality of anatomical regions, extracts at least one region from the plurality of anatomical regions, calculates a radiation dose index value for the radiography of the extracted region, based on a pixel value in the extracted region.

**F I G.  1**

## Description

TECHNICAL FIELD

The present invention relates to an image processing technique for outputting a radiation dose index value based on an image obtained through radiography

BACKGROUND ART

Recent years have seen growing use of digital images in the medical field. Using a digital radiographic apparatus (radiation shooting apparatus) that uses a sensor for indirectly or directly converting radiation (X-rays) into electrical signals in order to generate a digital image has been a main trend.

This radiographic apparatus has a very wide dynamic range for a radiation dose, and also has an advantage that, even when there is a deficiency or excess in the radiation dose, stable-density output is obtained through automatic density correction that is performed through image processing, compared with conventional analog radiography. On the other hand, even when a radiographing operator performs shooting with an inappropriate radiation dose, he or she is unlikely to notice that, and, in particular, when the radiation dose is excessive, there is the issue that a patient's exposure amount increases.

In view of this, in order to solve this issue, a value that is a standard of a radiation dose for shooting a digital radiological image (hereinafter, referred to as a "radiation dose index value") is usually displayed along with a shot image. In addition, various methods for calculating a radiation dose index value have been proposed. Recently, the international standard IEC62494-1 was issued by IEC (International Electrotechnical Commission), and EI (Exposure Index) was defined as a standardized radiation dose index value. In addition, in this international standard, EIT (Target Exposure Index) is determined as the value of a radiation dose (hereinafter, referred to as a "radiation dose target value") that is to be a target, and a method for conducting radiation dose management using DI (Deviation Index) indicating the difference between the radiation dose index value EI and the radiation dose target value EIT is also provided. Manufacturers provide radiation dose management functions conforming to this international standard. Manufacturers have proposed various calculation methods such as those in Patent Documents 1 and 2.

CITATION LIST

PATENT LITERATURE

PTL1: Japanese Patent Laid-Open No.

2014-158580
PTL1: Japanese Patent Laid-Open No. 2015-213546

SUMMARY OF INVENTION

TECHNICAL PROBLEM

A method for calculating a radiation dose index value EI has been a black box in most cases. Therefore, numerical implication of the radiation dose index value EI is not clear to the operator, and has been inconvenient when used as a reference value of radiation dose management.

This disclosure provides a technique for performing more appropriate radiation dose management using a reference intended by the operator.

SOLUTION TO PROBLEM

An image processing apparatus according to the present invention has the following configuration as means for achieving the above purpose. Specifically, the image processing apparatus includes division means for dividing a radiological image obtained by performing radiography on a subject, into a plurality of anatomical regions, extraction means for extracting at least one region from the plurality of anatomical regions, and calculation means for calculating a radiation dose index value of the radiography to be performed on the extracted region, based on a pixel value in the region extracted by the extraction means.

ADVANTAGEOUS EFFECTS OF INVENTION

According to the present invention, it is possible to perform more appropriate radiation dose management using a reference intended by the operator.

Other features and advantages of the present invention will be apparent from the following description taken in conjunction with the accompanying drawings. Note that the same reference numerals denote the same or like components throughout the accompanying drawings.

BRIEF DESCRIPTION OF DRAWINGS

The accompanying drawings, which are incorporated in and constitute a part of the specification, illustrate embodiments of the invention and, together with the description, serve to explain principles of the invention.

FIG. 1 shows a configuration example of a radiographic apparatus according to a first embodiment.
FIG. 2 is a flowchart showing the processing procedure of a radiographic apparatus 100 according to the first embodiment.
FIG. 3 is a flowchart showing the processing procedure for changing a region for calculating a radiation dose index value according to the first embodiment.

FIG. 4 shows a configuration example of the radiographic apparatus according to the first embodiment.

FIG. 5 is a flowchart showing the processing procedure for changing a division configuration for a region for calculating a radiation dose index value according to a second embodiment.

FIG. 6 shows a configuration example of a radiographic apparatus according to a third embodiment.

FIG. 7 is a flowchart showing the processing procedure for automatically updating a radiation dose target value EIT according to the third embodiment.

FIG. 8A is a diagram showing an example of a segmentation map according to the first embodiment.

FIG. 8B is a diagram showing an example of a changed correct-answer segmentation map according to the second embodiment.

FIG. 9 is a diagram showing an example of a correspondence table of the correspondence between a plurality of shooting sites and label numbers.

DESCRIPTION OF EMBODIMENTS

The present invention will be described in detail below with reference to the accompanying drawings based on exemplary embodiments. Note that configurations described in the following embodiments are merely exemplary, and the present invention is not limited to the illustrated configurations.

First Embodiment

Configuration of Radiographic Apparatus

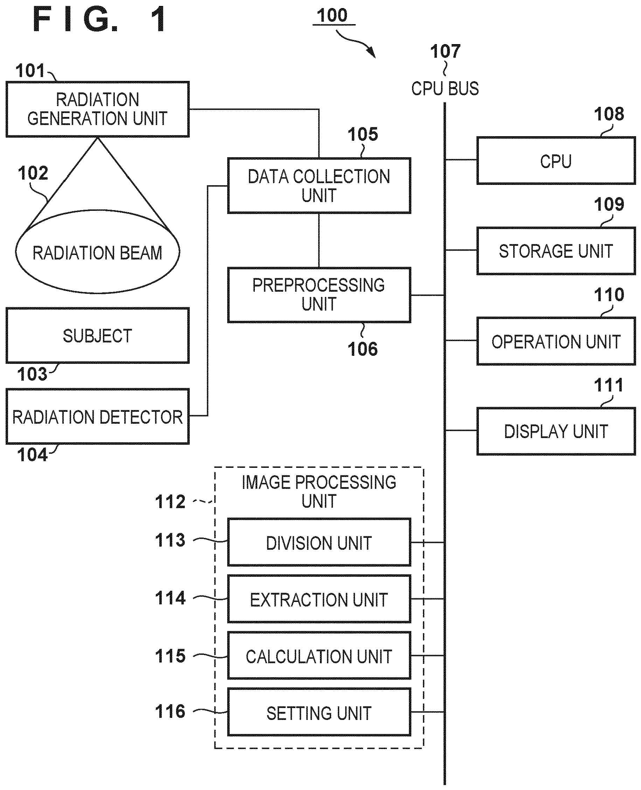

FIG. 1 shows a configuration example of a radiographic apparatus 100 according to a first embodiment. The radiographic apparatus 100 is a radiographic apparatus that has an image processing function for outputting a radiation dose index value based on an image obtained through radiography. Accordingly, the radiographic apparatus 100 also functions as an image processing apparatus. The radiographic apparatus 100 includes a radiation generation unit 101, a radiation detector 104, a data collection unit 105, a preprocessing unit 106, a CPU (Central Processing Unit) 108, a storage unit 109, an operation unit 110, a display unit 111, and an image processing unit 112, and these units are connected such that data can be mutually transmitted/received via a CPU bus 107. In addition, the image processing unit 112 includes a division unit 113, an extraction unit 114, a calculation unit 115, and a setting unit 116. The storage unit 109 stores various types of data and control programs required for processing to be performed by the CPU 108, and functions as a working memory of the CPU 108. The CPU 108 performs operation control and the like of the entire apparatus in accordance with an operation from the operation unit 110, using the storage unit 109. Accordingly, the radiographic apparatus 100 operates in a manner to be described later.

First, when a shooting instruction is input by the operator via the operation unit 110, this shooting instruction is transmitted to the radiation generation unit 101 and the radiation detector 104 via the data collection unit 105 by the CPU 108. Subsequently, the CPU 108 controls the radiation generation unit 101 and the radiation detector 104 to execute radiography. In the radiography, first, the radiation generation unit 101 irradiates a subject 103 with a radiation beam 102. The radiation beam 102 emitted from the radiation generation unit 101 passes through the subject 103, and reaches the radiation detector 104. The radiation detector 104 then outputs signals that are based on the intensity (radiation intensity) of the radiation beam 102 that reached the radiation detector 104. Note that, according to this embodiment, the subject 103 is a human body. Thus, the signals that are output from the radiation detector 104 are data obtained by shooting a human body.

The data collection unit 105 converts the signals output from the radiation detector 104 into predetermined digital signals, and supplies the digital signals as image data to the preprocessing unit 106. The preprocessing unit 106 performs preprocessing such as offset correction and gain correction on the image data supplied from the data collection unit 105. The image data subjected to preprocessing by the preprocessing unit 106 is sequentially transferred to the storage unit 109 and the image processing unit 112 via the CPU bus 107 under control by the CPU 108.

The image processing unit 112 performs processing for calculating a radiation dose index value based on the image data (hereinafter, "radiological image") obtained from the preprocessing unit 106. The radiation dose index value is a value that is a standard of a radiation dose for shooting as described above. The division unit 113 divides the radiological image (radiological image obtained by performing radiography on the subject 103) that has been input thereto, into a plurality of anatomical regions. According to this embodiment, the division unit 113 creates a segmentation map (multivalued image) to be described later. The extraction unit 114 extracts at least one region from among the plurality of anatomical regions divided by the division unit 113, as a region for calculating a radiation dose index value, based on an operator's operation on the operation unit 110. The calculation unit 115 calculates a radiation dose index value for performing radiography on the region extracted by the extraction unit 114, based on pixel values in the extracted region. The setting unit 116 sets and manages information regarding the correspondence between label number and training data or shooting site, which will be described later.

The radiation dose index value calculated by the image processing unit 112 is displayed on the display unit 111 along with the radiological image obtained from the preprocessing unit 106 under control by the CPU 108.

After the operator confirms the radiation dose index value and radiological image displayed on the display unit 111, a series of shooting operations end. The radiation dose index value and radiological image displayed on the display unit 111 may also be output to a printer or the like (not illustrated).

Operations of Radiographic Apparatus

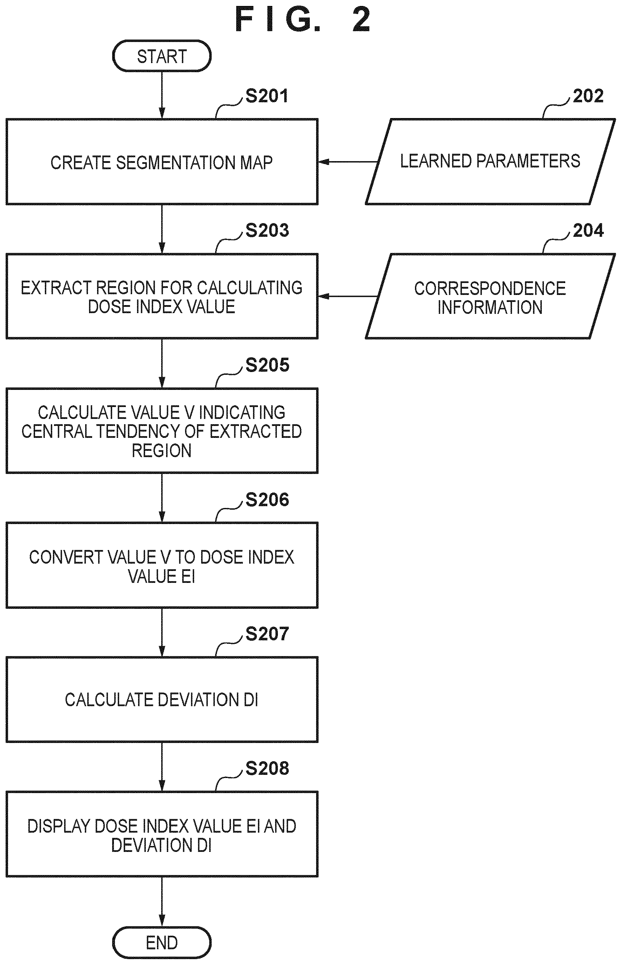

Next, operations of the radiographic apparatus 100 according to this embodiment will be described in detail with reference to the flowchart in FIG. 2. FIG. 2 is a flowchart showing the processing procedure of the radiographic apparatus 100 according to this embodiment. The flowchart shown in FIG. 2 can be realized as a result of the CPU 108 executing a control program stored in the storage unit 109, and executing computation and processing of information as well as control of items of hardware.

As described above, the radiological image obtained by the preprocessing unit 106 is transferred to the image processing unit 112 via the CPU bus 107. The transferred radiological image is input to the division unit 113. The division unit 113 creates a segmentation map (multivalued image) from the input radiological image (step S201). Specifically, the division unit 113 adds, to each pixel of the radiological image, a label indicating an anatomical region to which that pixel belongs. Such division of an image into any anatomical regions is called semantic segmentation (semantic region division). Note that, according to this embodiment, the label is a label number distinguishable by the pixel value. Specifically, the division unit 113 divides a radiological image into a plurality of anatomical regions by adding different label numbers to the plurality of anatomical regions.

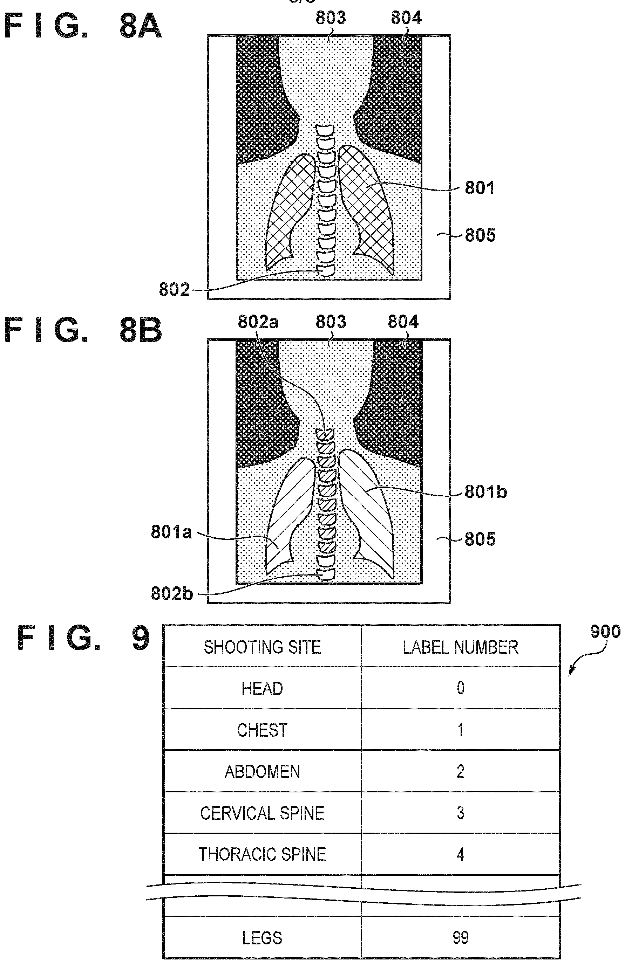

FIG. 8A shows an example of the segmentation map created in step S201. FIG. 8A is a diagram showing an example of a segmentation map when an input radiological image is an image that shows a chest. As shown in FIG. 8A, the division unit 113 provides the same value (e.g., a pixel value of 0) to the pixels of a region that belongs to a lung field 801, and provides the same value (e.g., a pixel value of 1) different from the above value of the lung field to the pixels of a region that belongs to a spine 802. In addition, the division unit 113 provides the same value (e.g., a pixel value of 2) different from the above values of the lung field and spine to the pixels that belong to a subject structure 803 that does not belong to any of the lung field 801 and the spine 802.

Note that division shown in FIG. 8A is exemplary, and the granularity according to which a radiological image is divided into anatomical regions is not limited in particular. For example, the division unit 113 may determine the granularity of division as appropriate according to a desired region for calculating a radiation dose index value instructed by the operator via the operation unit 110. In addition, the division unit 113 may also create a segmentation map by adding label numbers (pixel values) to regions other than the subject structure in a similar manner. For example, in FIG. 8A, it is also possible to create a segmentation map in which different label numbers are added to a region 804 in which radiation directly reaches the radiation detector 104 and a region 805 in which radiation is shielded by a collimator (illustrated).

According to this embodiment, the division unit 113 creates a segmentation map in step S201 using a known method. Here, a CNN (Convolutional Neural Network) is used as an example of the known method. A CNN is a neural network constituted by a convolution layer, pooling layer, full-connected layer, and the like, and is realized by appropriately combining such layers according to a problem to be solved. In addition, a CNN represents a type of a machine learning algorism, and requires prior training. Specifically, a filter coefficient used for a convolutional layer and parameters (variables) such as a weight and bias value of each layer need to be adjusted (optimized) through so-called supervised learning that uses a large number of pieces of training data.

Here, supervised learning includes preparation of a large number of samples of combination of an image that is input to the CNN (input image) and an output result (correct answer) expected when that input image is provided, and repeated adjustment of parameters so as to output an expected result. The error backpropagation method (back propagation) is commonly used for this adjustment. Specifically, parameters of the CNN are repeatedly adjusted in a direction in which the difference between the correct answer and actual output result (error defined by a loss function) decreases.

Note that, according to this embodiment, an image that is input to the CNN is a radiological image obtained by the preprocessing unit 106, and an expected output result is a correct-answer segmentation map (e.g., FIG. 8A). Such a correct-answer segmentation map can be created by the operator in accordance with the granularity of a desired anatomical region in advance. CNN parameters (learned parameters 202) are generated in advance through machine learning using a plurality of samples of combination of an input image and an expected output result. The learned parameters 202 are stored in the storage unit 109 in advance, and, when the division unit 113 creates a segmentation map in step S201, the learned parameters 202 are called, and semantic segmentation is performed using the CNN Accordingly, the division unit 113 divides the radiological image into a plurality of anatomical regions using parameters generated through machine learning in advance. In addition, the operator can determine, as training data, predetermined image data and a correct-answer segmentation map corresponding to the predetermined image data (division/allocation data). The training data is managed by the setting unit 116.

Note that only one learned parameter 202 may also be generated using data of all of the sites, but a learned parameter 202 may also be generated for each site (e.g., head, chest, abdomen, four extremities). Specifically, a configuration may also be adopted in which learning is separately performed using a plurality of samples of combinations of an input image and an expected output result for each site, and thereby a plurality of sets of learned parameters 202 are generated for each site. A configuration may also be adopted in which, when a plurality of sets of learned parameters 202 are generated, learned parameters of each set are stored in the storage unit 109 in advance in association with site information, and the division unit 113 calls learned parameters 202 corresponding to a shooting site from the storage unit 109 according to a site of an input image, and perform semantic segmentation using the CNN

Note that the network structure of the CNN is not particularly limited, and a generally known structure may be used. Specifically, FCN (Fully Convolutional Networks), SegNet, U-net, or the like can be used for machine learning. In addition, according to this embodiment, an image that is input to the CNN is a radiological image obtained by the preprocessing unit 106, but a radiological image obtained by reducing such a radiological image may also be used as an image that is input to the CNN Semantic segmentation that uses a CNN requires a large calculation amount and a long calculation time, and thus use of reduced image data can lead to a reduction in the calculation time.

Next, the extraction unit 114 extracts a region for calculating a radiation dose index value (step S203). Specifically, the extraction unit 114 extracts, as a region for calculating a radiation dose index value, a region specified by the operator via the operation unit 110. According to this embodiment, in order to perform the extraction processing, correspondence information 204 that is information regarding the correspondence between a shooting site and a label number is used as region information. Specifically, the extraction unit 114 extracts at least one region out of a plurality of anatomical regions using the correspondence information 204 that is information regarding the correspondence between a plurality of sites and label numbers respectively corresponding to the plurality of sites, in accordance with an operator's instruction, the correspondence information 204 having been set in advance. FIG. 9 shows, as an example of the correspondence information 204, an example of a table of the correspondence between a plurality of shooting sites and label numbers respectively corresponding to the plurality of sites. A correspondence table 900 shown in FIG. 9 is created by the operator or the like in advance, and is stored in the storage unit 109. The extraction unit 114 references the correspondence table 900, and obtains a label number corresponding to the site of a region designated (instructed) by the operator via the operation unit 110. Note that the extraction unit 114 may also directly obtain a label number specified by the operator via the operation unit 110. Subsequently, the extraction unit 114 generates a mask image (Mask) in which the value of a pixel corresponding to the obtained label number is 1, based on Expression 1.

$$\mathrm{Mask(x,y)} = \begin{cases} 1, & \mathrm{Map(x,y) = L} \\ 0, & \mathrm{Map(x,y) \neq L} \end{cases} \quad \ldots (1)$$

Note that Map indicates a segmentation map, and (x,y) indicates coordinates in an image. Also, L indicates an obtained label number.

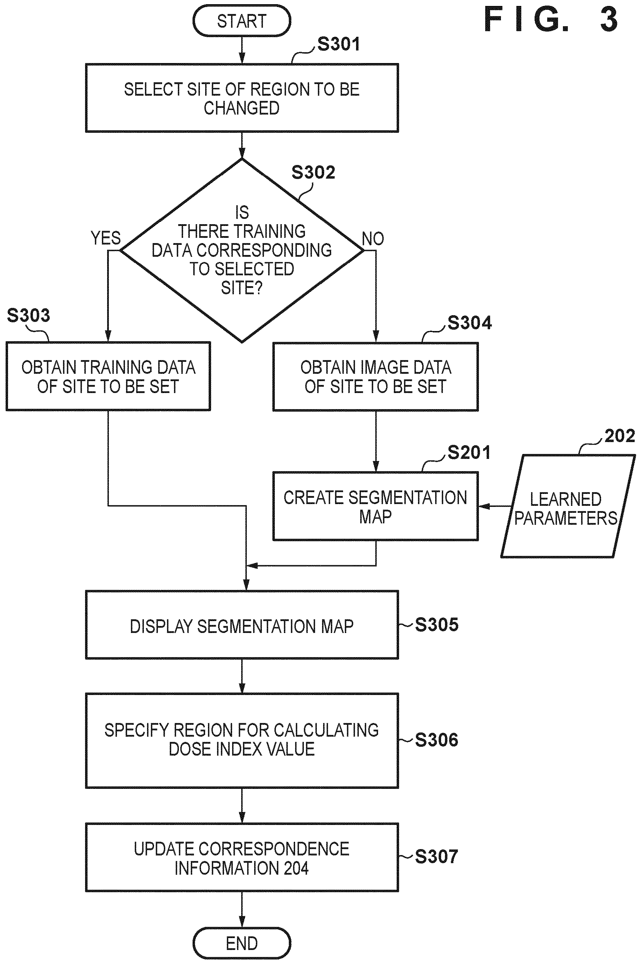

Note that the number of regions specified by the operator is not limited to one, and a plurality of regions may also be specified. When the operator specifies a plurality of regions, the extraction unit 114 may generate a mask image in which the value of a pixel corresponding to one of a plurality of label numbers corresponding to a plurality of region is 1, and the value other than that is 0. Note that a method for the operator to set a region will be described in detail later with reference to the flowchart in the FIG. 3.

Next, the calculation unit 115 calculates a value V indicating the central tendency of the extracted region, as a representative value in the region extracted in step S203 (i.e., a region in the mask image in which the pixel value is 1) (step S205). According to this embodiment, the value V is calculated as in Expression 2.

$$V = \frac{\sum_x \sum_y \mathrm{Org(x,y) \cdot Mask(x,y)}}{\sum_x \sum_y \mathrm{Mask(x,y)}} \quad \ldots (2)$$

Note that Org indicates an input image (according to this embodiment, a radiological image obtained by the preprocessing unit 106), Mask indicates a mask image, (x,y) indicates coordinates in the image, and Org (x, y) indicates a pixel value at coordinates (x,y) in the input image.

Next, the calculation unit 115 converts the obtained value V into a radiation dose index value EI (step S206). Specifically, the calculation unit 115 converts the value V into a radiation dose index value EI in accordance with the definition of international standard IEC62494-1 as in Expression 3.

$$\mathrm{EI} = c_0 \cdot g(V), \quad c_0 = 100\mu\mathrm{Gy}^{-1} \quad \ldots (3)$$

Note that a function g is a function for converting the value V into air kerma, and is determined in advance in accordance with the relationship between the air kerma and the value V under a stipulated condition. Note that the function g differs according to the property of the radiation detector 104. Therefore, the operator stores a plurality of functions g in the storage unit 109 in advance in correspondence with available radiation detectors 104, such that the calculation unit 115 can perform conversion using a function g corresponding to a radiation detector 104 that is actually used.

In addition, the calculation unit 115 calculates the difference amount (deviation) DI between a radiation dose target value EIT and the radiation dose index value EI, using Expression 4 (step S207).

$$DI = 10 \cdot \log_{10}\left(\frac{EI}{EI_T}\right) \qquad \ldots (4)$$

Note that the radiation dose target value EIT is set in advance for each site in accordance with a radiation dose management reference determined by the operator. The deviation DI is a numerical value indicating the deviation between the radiation dose target value EIT and the radiation dose index value EI, and thus, if the radiation dose target value EIT and the radiation dose index value EI are the same, the deviation DI is 0. In addition, the larger the radiation dose index value EI is, in other words the larger the extent to which the radiation dose of a shot image is larger than the radiation dose target value EIT, the larger the deviation DI becomes. For example, when the radiation dose of a shot image is twice as large as the radiation dose target value EIT, the deviation DI is about 3. In addition, the smaller the extent to which the radiation dose of a shot image is smaller than the radiation dose target value EIT, the smaller the deviation DI becomes, and, for example, when the radiation dose of a shot image is half the radiation dose target value, the deviation DI is about -3. Accordingly, the operator can instantaneously understand whether the radiation dose of a shot image is deficient or excessive relative to the radiation dose target value EIT that is a reference.

Next, the CPU 108 displays the obtained the radiation dose index value EI and deviation DI, on the display unit 111 (step S208). Here, the display method is not particularly limited, and, for example, the CPU 108 can perform the display on the display unit 111 along with the radiological image obtained by the preprocessing unit 106, and the CPU 108 may also perform control so as to display the obtained radiation dose index value EI and deviation DI as an overlay on a portion of the display area on which the display unit 111 can perform display.

Note that, according to this embodiment, only one radiation dose index value EI and only one deviation DI are calculated based on a region specified by the operator, but, for example, a configuration may also be adopted in which a plurality of radiation dose index values EI and deviations DI are obtained. Specifically, a configuration may also be adopted in which, when a plurality of regions are extracted as a result of the operator specifying these regions, the radiographic apparatus 100 calculates a value D for each of the regions, calculates the radiation dose index value EI and the deviation DI for the value D, and display such data on the display unit 111.

An operation of calculating a radiation dose index value based on a radiological image obtained through shooting has been described above. Next, operations of the image processing unit 112 when the operator changes a region for calculating a radiation dose index value will be described with reference to the flowchart in FIG. 3. FIG. 3 is a flowchart showing the processing procedure for changing a region for calculating a radiation dose index value. The flowchart shown in FIG. 3 can be realized as a result of the CPU 108 executing a control program stored in the storage unit 109, and executing computation and processing of information as well as control of each item of hardware. Note that, in the flowchart in FIG. 3, the same reference signs are assigned to the same steps as the steps shown in FIG. 2, and a description thereof is omitted.

First, the operator selects the site of a region (site to which this region belongs) to be changed via the operation unit 110 (step S301). Next, the setting unit 116 determines whether or not there is training data corresponding to the selected site (step S302). Here, as described above, training data refers to a correct-answer segmentation map determined by the operator (division/allocation data). If there is training data corresponding to the selected site (Yes in step S302), the setting unit 116 obtains the training data from the storage unit 109 (step S303). If there is no training data corresponding to the selected site (No in step S302), the setting unit 116 obtains, from the storage unit 109, image data (radiological image) of the selected site obtained through past shooting (step S304), and the division unit 113 creates a segmentation map from the obtained image data (step S201). A method for creating a segmentation map is the same as that in the description on step S201 in FIG. 2.

Next, the CPU 108 displays a segmentation map such as that shown in FIG. 8A, on the display unit 111 (step S305). The operator specifies a region to be changed, which is any region for calculating a radiation dose index value, using a mouse or the like (not illustrated) (step S306). Next, the setting unit 116 obtains a label number corresponding to the specified region, updates the correspondence information 204 (e.g., the correspondence table 900 of the correspondence between shooting sites and label numbers shown in FIG. 9), and stores (sets) the data in the storage unit 109 (step S307).

As described above, according to the first embodiment, it is possible to freely change a region for calculating a radiation dose index value from among regions obtained by dividing a radiological image, and to perform appropriate radiation dose management using a reference intended by the operator.

Second Embodiment

Configuration of Radiographic Apparatus

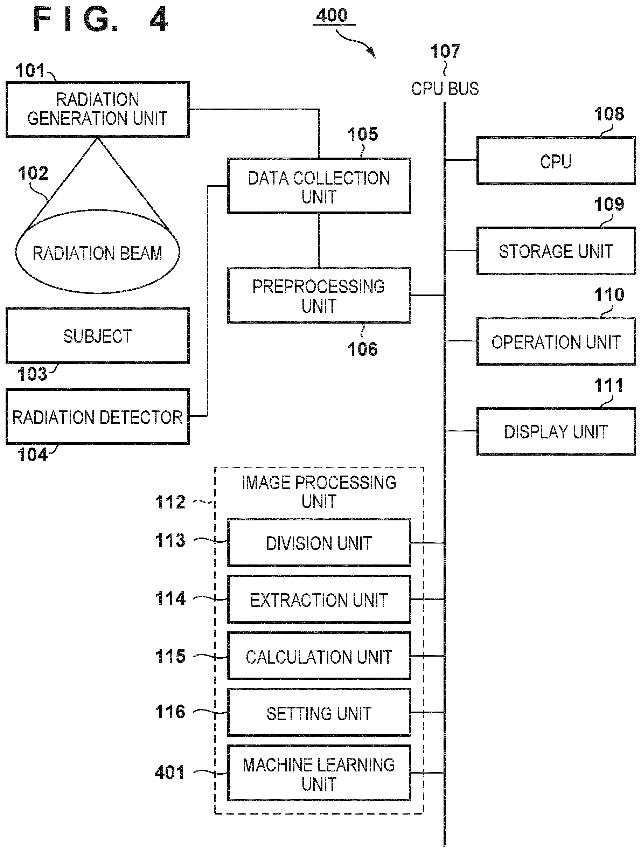

FIG. 4 shows a configuration example of a radiographic apparatus 400 according to a second embodiment. The radiographic apparatus 400 has a configuration of the radiographic apparatus 100 shown in FIG. 1 that includes a machine learning unit 401. The machine learning unit 401 has a function of performing leaning for changing a division configuration for a region for calculating a radiation dose index value. Specifically, the machine learning unit 401 performs machine learning (CNN relearning) based on training data (predetermined image data and a correct-answer segmentation map (division/allocation data) corresponding to the predetermined image data).

Operations of Radiographic Apparatus

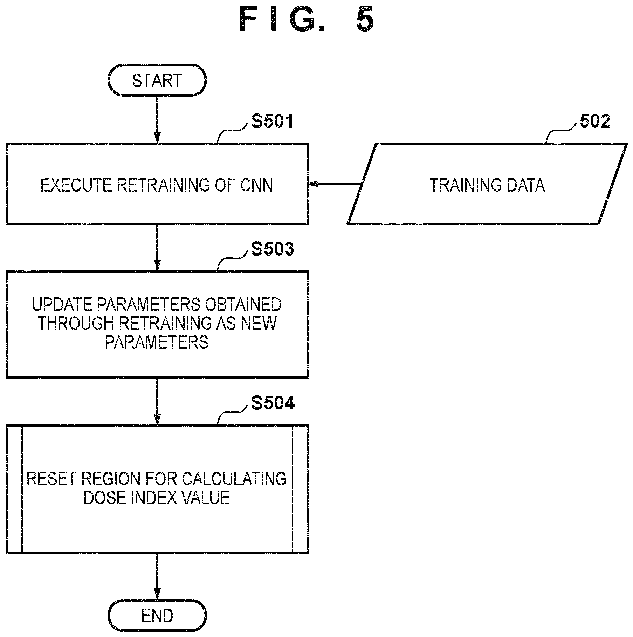

Processing for changing the division configuration for a region for calculating a radiation dose index value, which is an operation different from that in the first embodiment, will be described below with reference to FIG. 5. FIG. 5 is a flowchart showing the processing procedure for changing the division configuration for a region for calculating a radiation dose index value. The flowchart shown in FIG. 5 can be realized as a result of the CPU 108 executing a control program stored in the storage unit 109, and executing computation and processing of information as well as control of each item of hardware.

First, the machine learning unit 401 retrains the CNN based on training data 502 (step S501). This retraining is training that uses the training data 502 prepared in advance. Note that a specific training method is performed by repeatedly adjusting parameters of the CNN in a direction in which the difference between the correct answer and actual output result (error defined by a loss function) decreases, using the error backpropagation method (back propagation) similarly to that described in the first embodiment.

Here, the setting unit 116 can set training data for the machine learning unit 401 to perform retraining, as will be described below. Specifically, the setting unit 116 can change the correct-answer segmentation map that is training data, and set the correct-answer segmentation map in the machine learning unit 401. FIG. 8B shows an example of the changed correct-answer segmentation map. For example, according to the first embodiment, the division unit 113 adds the same label to the lung field 801 as one region as shown in FIG. 8A. On the other hand, according to this embodiment, as shown in FIG. 8B, the setting unit 116 prepares, as the training data 502, a correct-answer segmentation map in which different labels are added to a right lung field 801a and a left lung field 801b, in consideration of a case where the lung field is divided into different regions, namely a right lung field and a left lung field. In addition, according to the first embodiment, the division unit 113 adds the same label to the spine 802 as one region, as shown in FIG. 8A. On the other hand, according to this embodiment, the setting unit 116 prepares a correct-answer segmentation map in which different labels are added to a dorsal vertebra 802a and a lumbar vertebra 802b as shown in FIG. 8B, in consideration of a case where the spine is divided into a dorsal vertebra and a lumbar vertebra.

Next, the machine learning unit 401 updates (stores) parameters obtained through retraining as new parameters of the CNN, in the storage unit 109 (step S503). Subsequently, the image processing unit 112 resets a region for calculating a radiation dose index value (step S504). Here, the resetting method is the same as in the operation of the flowchart in FIG. 3, and thus a description thereof is omitted. Note that, in FIG. 3, as a result of using new training data in the above processing, a new segmentation map is created, and, in the process in step S307, the correspondence information 204 (e.g., the correspondence table 900 of the correspondence between shooting sites and label numbers shown in FIG. 9) is updated. Specifically, the setting unit 116 updates and sets the correspondence information 204 according to a plurality of anatomical regions obtained through division using the updated parameters. Accordingly, for example, in shooting for the next time onward (FIG. 2), as a result of replacing the learned parameters 202 (FIG. 2) with the parameters updated in step S503, the division unit 113 can divide a radiological image into a plurality of anatomical regions using the updated parameters. Furthermore, it is possible to calculate a radiation dose index value for a newly defined region by using the correspondence information 204 updated according to this embodiment in place of the correspondence information 204 that is used in step S203.

As described above, according to the second embodiment, there are effects that it is possible to change a division configuration for a region for calculating a radiation dose index value, and the operator can freely change the region for calculating a radiation dose index value.

Third Embodiment

Configuration of Radiographic Apparatus

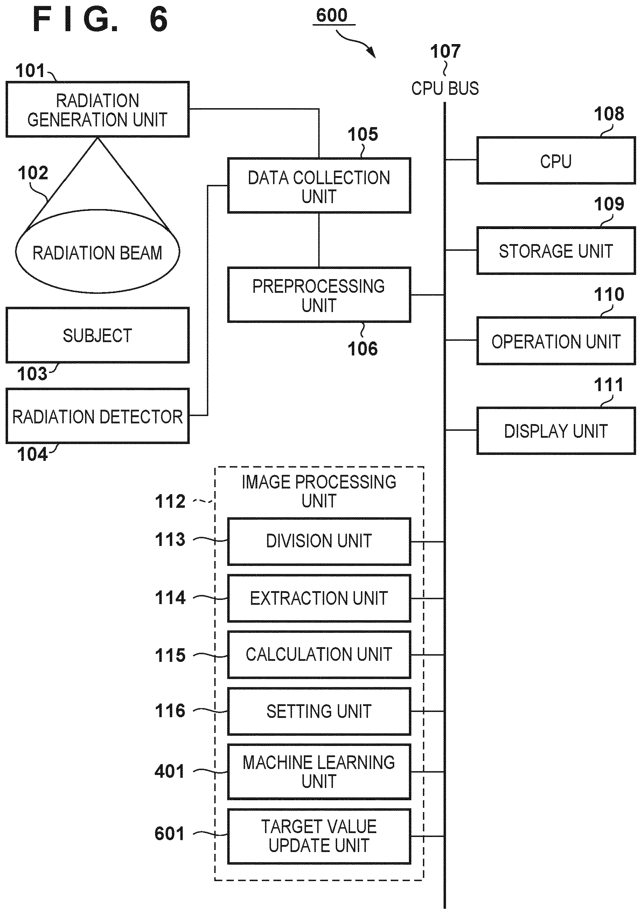

FIG. 6 shows a configuration example of a radiographic apparatus 600 according to a third embodiment. The radiographic apparatus 600 has a configuration of the radiographic apparatus 400 shown in FIG. 4 that includes a target value update unit 601. The target value update unit 601 has a function of automatically setting the radiation dose target value EIT.

The radiation dose target value EIT is a value that is a reference for the radiation dose index value EI, and, when a region for calculating the radiation dose index value EI changes, the radiation dose target value EIT also needs to be changed. This change is manually set by the operator in accordance with the radiation dose management reference, but, setting a region for calculating the radiation dose index value EI from scratch every time a region for calculating the radiation dose index value EI is changed is very troublesome. In view of this, the target value update unit 601 has a function for automatically updating the radiation dose target value EIT based on the difference of the region before and after change, using a value that is substantially equal to the radiation dose target value EIT before being changed.

Operation of Radiographic Apparatus

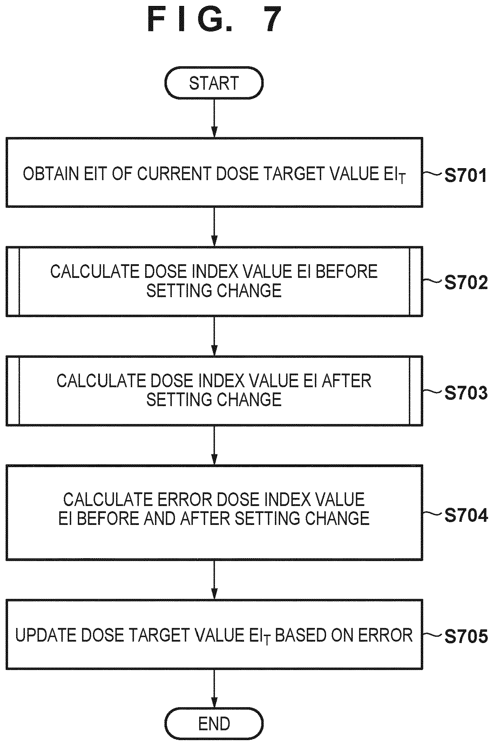

A method for automatically updating the radiation dose target value EIT will be described below with reference to FIG. 7. FIG. 7 is a flowchart showing the processing procedure for automatically updating the radiation dose target value EIT. The flowchart shown in FIG. 7 can be realized as a result of the CPU 108 executing a control program stored in the storage unit 109, and executing computation and processing of information as well as control of each item of hardware. Note that this flowchart is performed at a timing when a region for calculating a radiation dose target value is changed. Specifically, this flowchart is executed after when the region for calculating a radiation dose index value is changed (operation that is based on the flowchart in FIG. 3), or the division configuration for a region for calculating a radiation dose index value is changed (operation that is based on the flowchart in FIG. 5).

First, the target value update unit 601 obtains EIT that is currently set for a region for calculating a radiation dose target value (step S701). Next, the calculation unit 115 loads, from the storage unit 109, a plurality of radiological images obtained through past shooting, and calculates the radiation dose index value EI for each of the radiological images (step S702). Here, a method for calculating the radiation dose index value EI is the same as that described with reference to the flowchart in FIG. 2. Note that the parameter 202 learned when calculating the radiation dose index value EI and the correspondence information 204 that are used are the parameter 202 learned when calculating the radiation dose index value EI and the correspondence information 204, which have not been subjected to a setting change (change in the region for calculating a radiation dose index value (FIG. 3) or change in the division configuration for a region for calculating a radiation dose index value (FIG. 5)). Next, the calculation unit 115 calculates the radiation dose index value EI subjected to a setting change similarly to step S702 (step S703). Difference from step S702 is that the learned parameter 202 and the correspondence information 204 after a setting change are used.

Next, an error Err of EI before and after a setting change is calculated based on Expression 5 (step S704).

$$ \text{Err} = \frac{1}{N} \cdot \sum_{k=1}^{N} \left( EI_1(k) - EI_2(k) \right) \qquad \dots (5) $$

Note that k indicates an image number, and N indicates the total number of images for which EI was calculated. In addition, EI1 (k) and EI2 (k) are EIs calculated based on the image of the image number k, EI1 indicating EI before a setting change, and EI2 indicating EI after a setting change.

Next, the radiation dose target value EIT is updated using the obtained error Err (step S705).

$$ EI_T 2 = EI_T 1 + \text{Err} \qquad \dots (6) $$

Note that EIT1 indicates a radiation dose target value before update, and EIT2 indicates a radiation dose target value after update.

In this manner, when the learned parameter 202 is updated, or when the correspondence information 204 is updated, the target value update unit 601 updates a radiation dose target value that is a target value of a radiation dose index value, using radiation dose index values calculated by the calculation unit 115 before and after the update. As described above, according to the third embodiment, there is an effect that the trouble of the operator can be reduced by automatically updating the value of EIT during a setting change.

Although several embodiments have been described above, it is needless to say that the present invention is not limited to these embodiments, and various modifications and changes can be made within the scope of the gist.

(Other embodiments)

The present invention can be implemented by processing of supplying a program for implementing one or more functions of the above-described embodiments to a system or apparatus via a network or storage medium, and causing one or more processors in the computer of the system or apparatus to read out and execute the program. The present invention can also be implemented by a circuit (for example, an ASIC) for implementing one or more functions.

The present invention is not limited to the above embodiments and various changes and modifications can be made within the spirit and scope of the present invention. Therefore, to apprise the public of the scope of the present invention, the following claims are made.

This application claims priority from Japanese Patent Application No. 2018-151967 filed August 10, 2018, which is hereby incorporated by reference herein.

**Claims**

1. An image processing apparatus, **characterized by** comprising:

division means for dividing a radiological image obtained by performing radiography on a subject, into a plurality of anatomical regions; extraction means for extracting at least one region from the plurality of anatomical regions; and calculation means for calculating a radiation dose index value for the radiography of the region extracted by the extraction means, based on a pixel value in the extracted region.

2. The image processing apparatus according to claim 1, **characterized in that**
the division means adds different label numbers to the plurality of anatomical regions, and thereby divides a radiological image into a plurality of anatomical regions.

3. The image processing apparatus according to claim 1 or 2, **characterized in that**
the division means divides the radiological image into a plurality of anatomical regions using a parameter generated through machine learning in advance.

4. The image processing apparatus according to claim 3, **characterized by** further comprising:
storage means for storing a parameter generated through machine learning.

5. The image processing apparatus according to claim 4, **characterized in that**
the storage means stores the parameter generated through machine learning, in association with a site.

6. The image processing apparatus according to any one of claims 3 to 5, **characterized in that**
the extraction means extracts at least one region from the plurality of anatomical regions, using preset information regarding a correspondence between a plurality of sites and label numbers corresponding to the plurality of sites, in accordance with an operator's instruction.

7. The image processing apparatus according to claim 6, **characterized by** further comprising:

machine learning means for updating the parameter by performing machine learning based on predetermined image data and correct-answer division/allocation data corresponding to the predetermined image data, the predetermined image data and the correct-answer division/allocation data being set in advance, wherein the division means divides the radiological image into a plurality of anatomical regions using the updated parameter.

8. The image processing apparatus according to claim 7, **characterized by** further comprising:

setting means for updating and setting the correspondence information according to a plurality of anatomical regions obtained as a result of the division means dividing a radiological image using the updated parameter.

9. The image processing apparatus according to claim 8, **characterized by** further comprising:
update means for updating a radiation dose target value that is a target value of the radiation dose index value, using radiation dose index values calculated by the calculation means before and after the parameter or the correspondence information is updated.

10. The image processing apparatus according to any one of claims 3 to 9, **characterized in that**
the machine learning is machine learning that uses one of a CNN (Convolutional Neural Network), FCN (Fully Convolutional Networks), SegNet, and U-net.

11. The image processing apparatus according to any one of claims 1 to 10, **characterized in that**
the calculation means calculates, as a representative value, a value indicating a central tendency of the region in the radiological image extracted by the extraction means, and calculates the radiation dose index value using the representative value.

12. The image processing apparatus according to claim 11, **characterized in that**
when a plurality of regions are extracted from the plurality of anatomical regions by the extraction means,
the calculation means calculates representative values for the respective regions extracted by the extraction means, and calculates a plurality of radiation dose index values for the radiography of the plurality of extracted regions, based on the plurality of representative values.

13. An image processing method, **characterized by** comprising:

a division step of dividing a radiological image obtained by performing radiography on a subject, into a plurality of anatomical regions; an extraction step of extracting at least one region from the plurality of anatomical regions; and a calculation step of calculating a radiation dose index value for the radiography of the region extracted in the extraction step, based on a pixel value in the extracted region.

14. A program for causing a computer to function as the image processing apparatus according to any one of claims 1 to 12.

# FIG. 1

100

107
CPU BUS

**101**
RADIATION GENERATION UNIT

**102**
RADIATION BEAM

**103**
SUBJECT

**104**
RADIATION DETECTOR

**105**
DATA COLLECTION UNIT

**106**
PREPROCESSING UNIT

**108**
CPU

**109**
STORAGE UNIT

**110**
OPERATION UNIT

**111**
DISPLAY UNIT

**112** IMAGE PROCESSING UNIT

**113** DIVISION UNIT

**114** EXTRACTION UNIT

**115** CALCULATION UNIT

**116** SETTING UNIT

# FIG. 2

```
                    START

                      |
                      v
        ┌──────────────────────────┐  S201              S202
        │                          │           ┌──────────────────────────┐
        │  CREATE SEGMENTATION MAP │ <──────── │   LEARNED PARAMETERS     │
        │                          │           └──────────────────────────┘
        └──────────────────────────┘
                      |
                      v
        ┌──────────────────────────┐  S203              S204
        │ EXTRACT REGION FOR       │           ┌──────────────────────────┐
        │ CALCULATING              │ <──────── │   CORRESPONDENCE          │
        │ DOSE INDEX VALUE         │           │   INFORMATION             │
        └──────────────────────────┘           └──────────────────────────┘
                      |
                      v
        ┌──────────────────────────┐  S205
        │ CALCULATE VALUE V        │
        │ INDICATING CENTRAL       │
        │ TENDENCY OF EXTRACTED    │
        │ REGION                   │
        └──────────────────────────┘
                      |
                      v
        ┌──────────────────────────┐  S206
        │ CONVERT VALUE V TO DOSE  │
        │ INDEX VALUE EI           │
        └──────────────────────────┘
                      |
                      v
        ┌──────────────────────────┐  S207
        │ CALCULATE DEVIATION DI   │
        └──────────────────────────┘
                      |
                      v
        ┌──────────────────────────┐  S208
        │ DISPLAY DOSE INDEX VALUE │
        │ EI AND DEVIATION DI      │
        └──────────────────────────┘
                      |
                      v
                     END
```

# FIG. 3

```
                    ( START )
                        │
                        ▼  S301
        ┌───────────────────────────────┐
        │  SELECT SITE OF REGION TO BE   │
        │           CHANGED              │
        └───────────────────────────────┘
                        │
                        ▼        S302
                      ╱   ╲
            YES     ╱   IS   ╲     NO
         ◄─────────  THERE TRAINING ─────────►
                   ╲ DATA CORRESPONDING ╱
                    ╲ TO SELECTED ╱
                     ╲  SITE?  ╱
                       ╲    ╱
   S303                                     S304
   ┌─────────────────────┐        ┌─────────────────────┐
   │  OBTAIN TRAINING DATA│        │   OBTAIN IMAGE DATA  │
   │   OF SITE TO BE SET  │        │   OF SITE TO BE SET  │
   └─────────────────────┘        └─────────────────────┘
            │                               │
            │                               ▼    S201          202
            │                  ┌─────────────────────┐   ┌──────────────┐
            │                  │ CREATE SEGMENTATION  │◄──│   LEARNED    │
            │                  │        MAP           │   │  PARAMETERS  │
            │                  └─────────────────────┘   └──────────────┘
            │                               │
            └───────────────┬───────────────┘
                            ▼
        ┌───────────────────────────────┐
        │   DISPLAY SEGMENTATION MAP     │  S305
        └───────────────────────────────┘
                            │
                            ▼
        ┌───────────────────────────────┐
        │ SPECIFY REGION FOR CALCULATING │  S306
        │       DOSE INDEX VALUE         │
        └───────────────────────────────┘
                            │
                            ▼
        ┌───────────────────────────────┐
        │    UPDATE CORRESPONDENCE       │  S307
        │      INFORMATION 204           │
        └───────────────────────────────┘
                            │
                            ▼
                        (  END  )
```

# FIG. 4

**400**

**107**
CPU BUS

**101**
RADIATION GENERATION UNIT

**102**
RADIATION BEAM

**103**
SUBJECT

**104**
RADIATION DETECTOR

**105**
DATA COLLECTION UNIT

**106**
PREPROCESSING UNIT

**108**
CPU

**109**
STORAGE UNIT

**110**
OPERATION UNIT

**111**
DISPLAY UNIT

**112** — IMAGE PROCESSING UNIT

**113** — DIVISION UNIT

**114** — EXTRACTION UNIT

**115** — CALCULATION UNIT

**116** — SETTING UNIT

**401** — MACHINE LEARNING UNIT

# F I G. 5

```
        ┌─────────┐
        │  START  │
        └─────────┘
             │
             ▼                    S501                        502
   ┌───────────────────────┐              ┌──────────────────────────┐
   │ EXECUTE RETRAINING    │◄─────────────│     TRAINING DATA        │
   │        OF CNN         │              └──────────────────────────┘
   └───────────────────────┘
             │
             ▼                    S503
   ┌───────────────────────┐
   │ UPDATE PARAMETERS     │
   │ OBTAINED THROUGH      │
   │ RETRAINING AS NEW     │
   │ PARAMETERS            │
   └───────────────────────┘
             │
             ▼                    S504
   ┌───────────────────────┐
   │ │ RESET REGION FOR  │ │
   │ │ CALCULATING DOSE  │ │
   │ │ INDEX VALUE       │ │
   └───────────────────────┘
             │
             ▼
        ┌─────────┐
        │   END   │
        └─────────┘
```

# FIG. 6

**600**

**107**
CPU BUS

**101**
RADIATION GENERATION UNIT

**102**

RADIATION BEAM

**105**
DATA COLLECTION UNIT

**108**
CPU

**109**
STORAGE UNIT

SUBJECT

**103**

**106**
PREPROCESSING UNIT

**110**
OPERATION UNIT

**111**
DISPLAY UNIT

**104**
RADIATION DETECTOR

IMAGE PROCESSING UNIT

**112**

**113** DIVISION UNIT

**114** EXTRACTION UNIT

**115** CALCULATION UNIT

**116** SETTING UNIT

**401** MACHINE LEARNING UNIT

**601** TARGET VALUE UPDATE UNIT

# F I G. 7

```
        ┌─────────┐
        │  START  │
        └─────────┘
             │
             ▼
┌──────────────────────────────────────────┐
│ OBTAIN EIT OF CURRENT DOSE TARGET VALUE EI_T │──S701
└──────────────────────────────────────────┘
             │
             ▼
┌──────────────────────────────────────────┐
│  CALCULATE DOSE INDEX VALUE EI BEFORE     │──S702
│          SETTING CHANGE                   │
└──────────────────────────────────────────┘
             │
             ▼
┌──────────────────────────────────────────┐
│  CALCULATE DOSE INDEX VALUE EI AFTER      │──S703
│          SETTING CHANGE                   │
└──────────────────────────────────────────┘
             │
             ▼
┌──────────────────────────────────────────┐
│     CALCULATE ERROR DOSE INDEX VALUE      │──S704
│   EI BEFORE AND AFTER SETTING CHANGE      │
└──────────────────────────────────────────┘
             │
             ▼
┌──────────────────────────────────────────┐
│ UPDATE DOSE TARGET VALUE EI_T BASED ON ERROR │──S705
└──────────────────────────────────────────┘
             │
             ▼
        ┌─────────┐
        │   END   │
        └─────────┘
```

FIG. 8A

803    804

801

805

802

FIG. 8B

802a    803    804

801a

801b

805

802b

FIG. 9

| SHOOTING SITE | LABEL NUMBER |
|---|---|
| HEAD | 0 |
| CHEST | 1 |
| ABDOMEN | 2 |
| CERVICAL SPINE | 3 |
| THORACIC SPINE | 4 |
| LEGS | 99 |

900

## INTERNATIONAL SEARCH REPORT

| International application No. |
| --- |
| PCT/JP2019/024229 |

**A. CLASSIFICATION OF SUBJECT MATTER**
Int.Cl. A61B6/00(2006.01)i, G06T7/00(2017.01)i, G06T7/11(2017.01)i

According to International Patent Classification (IPC) or to both national classification and IPC

**B. FIELDS SEARCHED**

Minimum documentation searched (classification system followed by classification symbols)
Int.Cl. A61B5/00-5/01, A61B5/055, A61B6/00-6/14, G06T1/00, G06T7/00-7/90

Documentation searched other than minimum documentation to the extent that such documents are included in the fields searched
Published examined utility model applications of Japan       1922-1996
Published unexamined utility model applications of Japan     1971-2019
Registered utility model specifications of Japan             1996-2019
Published registered utility model applications of Japan     1994-2019

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
| --- | --- | --- |
| X<br>Y<br>A | US 2017/0245825 A1 (VARIAN MEDICAL SYSTEMS, INC.) 31 August 2017, paragraphs [0020], [0023], [0033], [0035], fig. 2 (Family: none) | 1, 11-14<br>2-8, 10<br>9 |
| Y | US 2016/0328855 A1 (SIEMENS AKTIENGESELLSCHAFT) 10 November 2016, paragraphs [0004], [0027], [0060], [0062], fig. 2, 10 & CN 106108925 A | 2-8, 10 |

☒ Further documents are listed in the continuation of Box C.    ☐ See patent family annex.

| | | |
| --- | --- | --- |
| * | Special categories of cited documents: | |
| "A" | document defining the general state of the art which is not considered to be of particular relevance | "T" later document published after the international filing date or priority date and not in conflict with the application but cited to understand the principle or theory underlying the invention |
| "E" | earlier application or patent but published on or after the international filing date | "X" document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" | document referring to an oral disclosure, use, exhibition or other means | |
| "P" | document published prior to the international filing date but later than the priority date claimed | "&" document member of the same patent family |

| Date of the actual completion of the international search | Date of mailing of the international search report |
| --- | --- |
| 04.09.2019 | 17.09.2019 |

| Name and mailing address of the ISA/ | Authorized officer |
| --- | --- |
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**

| International application No. |
| --- |
| PCT/JP2019/024229 |

C (Continuation).  DOCUMENTS CONSIDERED TO BE RELEVANT

| Category* | Citation of document, with indication, where appropriate, of the relevant passages | Relevant to claim No. |
| --- | --- | --- |
| Y | JP 2002-325754 A (CANON INC.) 12 November 2002, paragraphs [0001], [0023], [0030], [0043], [0044], [0048], [0050], fig. 1, 8-10 <br> & US 2003/0007674 A1, paragraphs [0001], [0056], [0069], [0091]-[0094], [0098], [0100], fig. 1, 8-10 | 3-8 |
| Y | US 2018/0061058 A1 (ELEKTA, INC.) 01 March 2018, paragraphs [0001], [0023], [0028] <br> & WO 2018/039368 A1 & EP 3504681 A1 & AU 2017315674 A1 & CN 109906470 A | 10 |

Form PCT/ISA/210 (continuation of second sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2014158580 A
- JP 2015213546 A
- JP 2018151967 A